# EUROPEAN PATENT APPLICATION

(11) **EP 2 198 773 A1**
(43) Date of publication of application: **23.06.2010**
(21) Application number: 08425802.9
(22) Date of filing: 18.12.2008
(51) Int. Cl.: A61B 5/00, A61B 5/0225, A61B 5/022

(54) **System and method for self-validation of arterial pressure measurements**

(71) Applicant: Mismed S.R.L., 09126 Cagliari (IT)
(72) Inventor: Vallascas, Rinaldo, 09129 Cagliari (IT)
(74) Representative: Borsano, Corrado

(57) **Abstract**

The invention relates to a method and a non-invasive appliance for measuring arterial pressure comprising stages in which the sensors are calibrated, with a customised self-validation of the oscillometric method by means of a simultaneous or deferred comparison with an auscultatory or direct measurement method, the use of one or two pressure sensors to record the pressure and derive the oscillometric signal, and the optional insertion of a further plethysmographic palpatory sensor with a view to obtaining data to process in order to arrive at a measurement using the sensor fusion technique.

## Description

### Field of application of the invention

This invention concerns a new, non-invasive method and a self-referential device for determining an individual's arterial pressure that can also be implemented in a multifunctional version for use for prevention and diagnosis in the medical field and in the veterinary sector. More in particular, the invention concerns a method and a device for measuring arterial pressure by means of a customised technique for the self-validation of the measuring system by comparing a direct or indirect method with the oscillometric method, and optionally including the application of the sensor fusion technique.

### State of the art

Arterial pressure measurement using non-invasive techniques has gone through three centuries of research and development. Currently-used indirect methods are based substantially on experiments conducted by Riva Rocci-Korotkoff and by Marey, who paved the way to such modern methods as the auscultatory and the oscillometric, which have relegated the manual palpation technique to a secondary role.

While the former, the auscultatory method, is being increasingly used as a standardised reference, also in parallel with the less well-tolerated direct measurement, the latter, oscillometric method, is based on proprietary algorithms that have never been formalised. Moreover, it seems that no proposals aiming to standardise the latter have been presented as yet.

There are consequently no standardised guidelines to be used for design purposes in the development of automated devices based on oscillometry. The analytical solutions are of a heuristic nature and not fully elucidated. The user therefore has no guarantee, other than the fact that the instrument may have been recommended by an organisation in the sector, based on experimental observations recorded by a third party.

In fact, the criteria for issuing such a recommendation are based on statistical comparisons drawn on measurements evaluated using the auscultatory method, which fail to take into account the real characterisation of the appliance by defining its intrinsic metrological parameters, as concerns repeatability and accuracy, for instance. It has become common practice to postulate and assume a universal correlation between the two distinct approaches, although this has never been formalised.

Elsewhere attempts have been made to promote the automation of the palpation method, sometimes forgetting that physiological signs are only repetitive, not periodical.

Arterial pressure is one of the most difficult quantities to measure precisely, whether invasive or non-invasive methods are used. That is why, we often have to content ourselves with considerable uncertainties and methodological approximations that are unacceptable in other settings and for other physical quantities.

Although a proper understanding of the pressure values is often important for a preliminary characterisation of a given individual's cardiovascular system, the indirect measurement protocol is not always respected in every detail: as concerns the rate of inflation and deflation, and the relationship between the sizes of the arm and the armband, and the force used to wrap the armband around the arm, and the shape and stiffness of the armband; all this even without taking into account the never mentioned but nonetheless often far from negligible pressure gradient that should occur in the issue between the artery and the armband at the time of the measurement, which systematically makes the reading overestimate the real pressure.

It is also true, however, that other collateral problems, such as white coat hypertension and the clinical interpretation of single pressure measurements, could lead to the technical-scientific aspects taking second place in relation to those of a clinical nature. Nonetheless, there is no valid reason for underestimating any research and development efforts to achieve better systems and to establish more refined and suitable methods for reducing the measurement uncertainties.

It is also well known that the inaccuracy of systems based on oscillometry increases the extreme values (both high and low) of the quantities being measured and in particular situations, such as pregnancy and during physical activity.

For these reasons, international organisations such as the European Society of Hypertension (E. O'Brien, B. Weber, G. Parati J. Staessen, M.G. Myers, "Blood pressure-measuring devices: recommendations of the European Society of Hypertension" BMJ 2001, 322 pp 531-536; and the American Association for the Advancement of Medical Instrumentation have specified criteria for the validation and recommendation of automated arterial pressure measuring appliances that rely on the oscillometric method. Since 2004, there has also been a standard available, CEN 1060-4 "Test procedures to determine the overall system accuracy of automated non-invasive sphygmomanometers".

### Summary of the invention

The object of the present invention is therefore to make it unnecessary to implement experiments to achieve the validation needed for automatic arterial pressure monitors to be recommended by the organisations in the sector, by indicating a customised method for the self-validation of the oscillometric method applicable to individual patients, and a device that can optionally be operated using the sensor fusion technique in order to make the measurement even more "robust".

The qualifying feature of the invention refers to a customised self-tuning method that relies on a new methodological approach to the conduction of the measurement process applicable to the automated monitors used to determine mean, systolic and diastolic arterial pressures.

It also offers a further solution that involves making the systolic, mean and diastolic pressure measurements more robust by means of the logical interaction of different data obtained by a number of sensors that may vary up to three, depending on the application.

The self-tuning process involves performing a customised search for the correlation, for a given patient, between the oscillometric method and another method taken as a standard for reference, which may be a direct or indirect, auscultatory measurement technique, capable of providing a measure of the compatibility between the two techniques.

A particular object of the present invention is a method and a device for measuring arterial pressure by means of a customised technique for self-validation the measurement system by comparing a direct or indirect method with the oscillometric method, and optionally including the application of the sensor fusion technique, as described in more detail in the claims, which form an integral part of the present description.

### Brief description of the figures

Further objects and advantages of the present invention will emerge more clearly from the following detailed description of an embodiment, and variants thereof, and from the attached drawings, which are given as a non-limiting example, wherein:
- Figure 1 shows a first embodiment of a mean, systolic and diastolic pressure measuring device with an arm cuff;
- Figure 2 shows a second embodiment of a device for measuring mean, systolic and diastolic pressures with a finger cuff;
- Figure 3 shows a third embodiment of a device for measuring mean, systolic and diastolic pressures in animals;
- Figure 4 shows an embodiment of a multifunctional device for validating the oscillometric method that can be used both on a finger and on an arm, for measuring mean, systolic and diastolic pressures;
- Figure 5 shows time charts of recordings of oscillometric signals (below), on deflation and of the palpation signal obtained using the baro-plethysmographic method;
- Figure 6 shows time charts of recordings of oscillometric signals (below), on deflation and of the palpation signal obtained using the piezo-plethysmographic method.
- Figure 7 shows time charts of recordings of oscillometric signals (below), on deflation and of the palpation signal obtained with the photo-plethysmographic method.

In the figures, the same reference numbers identify the same elements or components.

### Detailed description

The various embodiments of the device forming the object of the invention are described below.

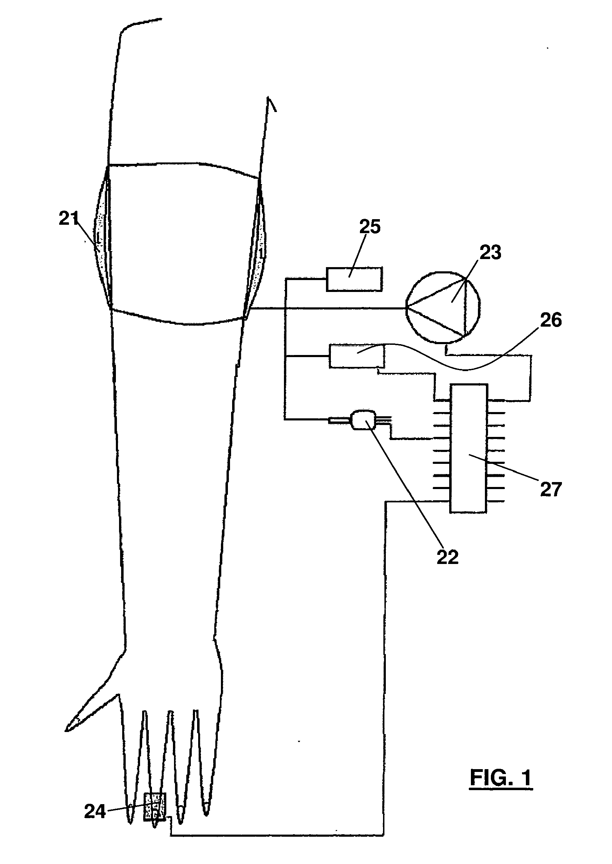

Figure 1 shows one of the possible embodiments of the system, wherein a cuff and the related oscillometric pressure sensor are applied to an arm, while a plethysmographic palpatory sensor is simultaneously attached to a finger of the same arm.

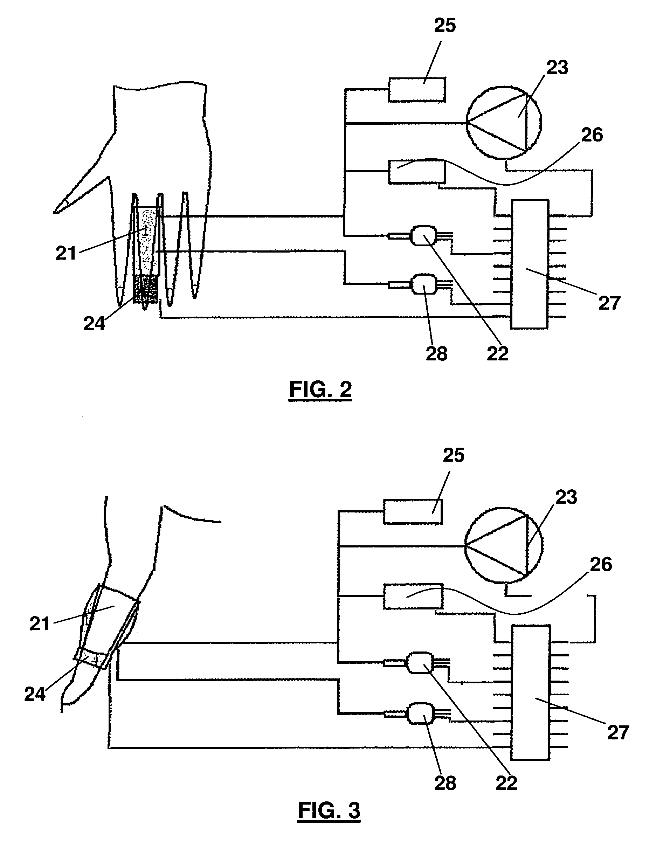

Figure 2 shows a second configuration of the system wherein the cuff is applied to a finger with a pressure sensor, with a second oscillometric sensor that uses a transmission fluid and, downstream therefrom, a plethysmographic palpatory sensor.

Figure 3 shows a third configuration wherein the cuff is applied to the limb of an animal, with an integrated plethysmographic palpatory sensor downstream therefrom.

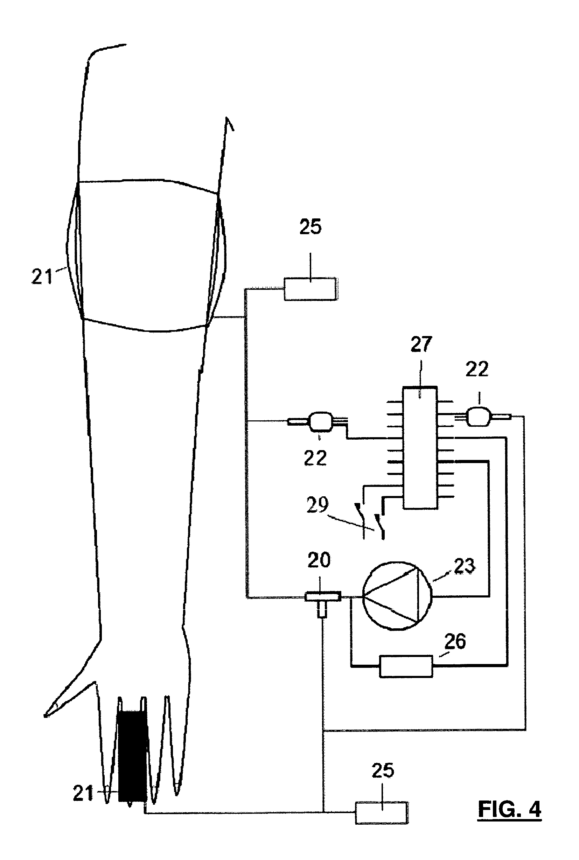

Figure 4 shows a multifunctional configuration that enables all the above-described validation and measurement functions to be achieved in one and the same device.

More in particular, with reference to figures 1, 2, 3 and 4, the appliance forming the object of the invention comprises the following devices of known type per se:
- a cuff 21, for applying to an arm (fig. 1 and 4), a second finger cuff 21' (fig. 2) and 21" (fig. 4); a cuff 21"' for applying around an animal's paw (fig. 3) (or also around its tail); given the numerous applications, the shape and size of these cuffs may vary, while their function remains exactly the same;
- a micro-compressor 23 for inflating the cuffs;
- a manually-adjustable or fixed deflation valve 25 for each cuff;
- a rapid deflation solenoid valve 26 for quickly releasing the pressure in the cuffs;
- an arterial pressure sensor 22 for recording the cuff inflation-deflation pressure;
- an optional three-way valve 20 (fig. 4), for selecting the inflation cuff, it being possible to select one or other, or both of the cuffs;
- an optional plethysmographic palpatory sensor 24 (fig. 1, 2, 3), applied to a finger, accompanied by a specific system for recording the plethysmographic palpatory signal; the sensor 24 may be of known type, as described later on;
- a further pressure transducer, or oscillometric sensor 28, preferably accompanied by an elastic probe, containing a fluid for transmitting the oscillometric signal picked up by the cuff 21, distinctly from the inflation/deflation pressure, preferably for use in the more critical applications, when the oscillometric signal is particularly low, e.g. for signals coming from finger cuffs;
- a microcontroller 27 for managing the information and controlling the operation of the above-described elements;
- an amplifier-filter system for determining the oscillograms, not shown in the figures;
- a display for viewing the processed data, not shown in the figures;
- resident software for data processing and calculations in the microcontroller 27;
- one or more switches 29 serving as a user interface, suitable for managing the system, which - during the self-tuning process described later on - enable the programme to be informed of the reaching of the systolic pressure (first switch) and diastolic pressure (second switch) in deflation, and vice versa in inflation, so that the individual parameters can be determined.

The plethysmographic palpatory signal sensor 24 may be made, for instance, using an electret membrane or an electromechanical film, or a resistive probe, or a piezoelectric probe, or a photo-plethysmographic probe, or a probe connected to a common pressure transducer by means of a transmission fluid.

Inside or downstream from the finger cuff 21, again in contact therewith, there may be a further elastic probe containing a fluid for improving the transmission of the oscillometric signal, and connected to a pressure transducer for determining said signal.

In practice, the micro-compressor 23 generates a pressure in the chamber of the cuff sufficient to determine the occlusion of the brachial artery (fig. 1 and fig. 4), or of the vascular system in the finger (fig. 2 and fig. 4) or animal's paw (or tail) (fig. 3). The deflation valve 25 enables the pressure to be released according to a virtually linear law.

During the deflation or inflation phases, the pressure sensor 22, possibly aided by the sensor 28, is used to record the trend of the pressure in the chamber of the cuff, and an oscillogram is obtained. At the same time, the sensor 24 is used to obtain an optional plethysmographic palpatory signal.

Interpreting the complete picture of the recordings obtained during the rising or falling pressure phase, enables the value of the three quantities of interest, i.e. the minimum, mean and maximum pressures, to be ascertained.

It would be preferable to operate in the inflation phase for various positive reasons concerning both the shorter total time it takes to complete the procedure and the more limited invasiveness of the method, minimising the dynamic effects of insertion, but this makes it necessary to "filter" the noise relating to the compressor's operation. So the measurements are in fact taken in the deflation phase in the examples discussed below.

With reference to figures 4, 5 and 6, examples are given of time charts recording the oscillometric signals, the deflation signal and the palpatory signal obtained using the device forming the object of the invention.

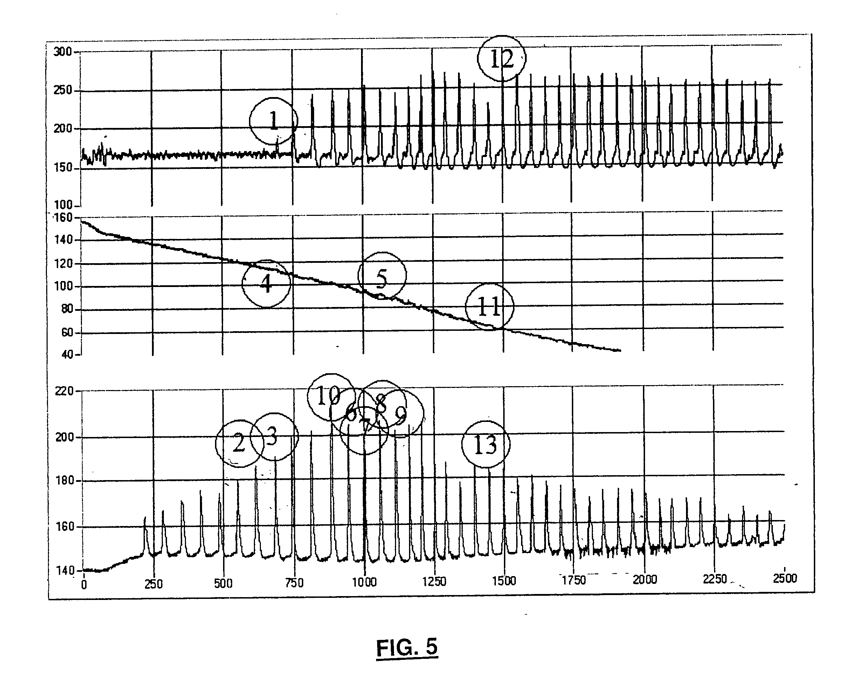

Figure 5 shows three graphs obtained by recording the signals from an arm cuff 21 and a finger sensor 24.
- At the top, there is the baro-plethysmographic palpatory signal obtained with the sensor 24, of the type involving a probe containing a fluid and communicating with a pressure transducer connected to the probe (not shown in the figures);
- in the middle, there is the signal relating to the pressure on deflation of the cuff 21, obtained by the pressure transducer 22;
- at the bottom, there is the classic oscillometric signal, obtained by the oscillometric sensor 28.

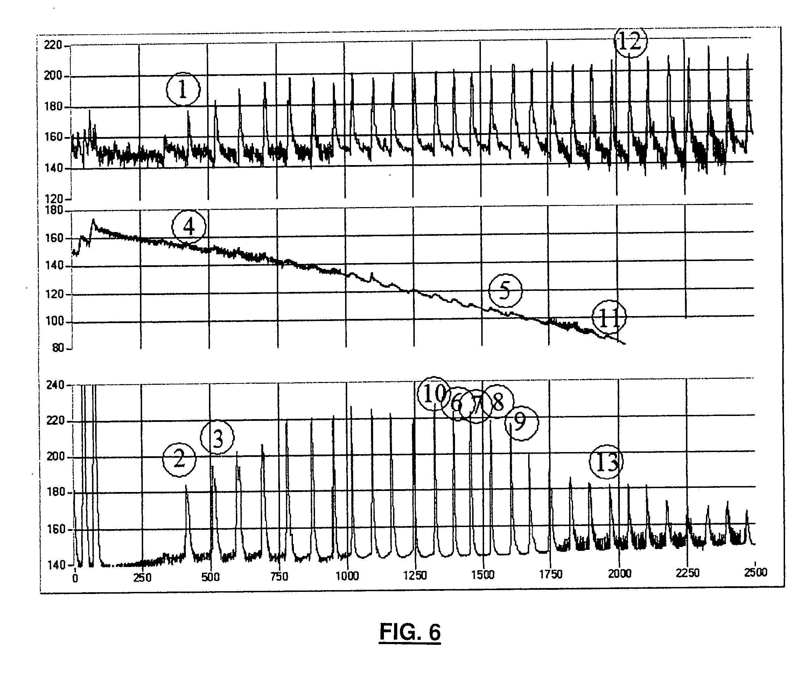

Figure 6 shows three graphs obtained by recording the signals from an arm cuff 21 and a finger sensor 24.
- At the top, there is the plethysmographic palpatory signal obtained with the sensor 24, of a type involving a piezoelectric probe;
- in the middle, there is the signal relating to the pressure on deflation of the cuff 21, obtained by the pressure transducer 22;
- at the bottom, there is the classic oscillometric signal, obtained by the oscillometric sensor 28.

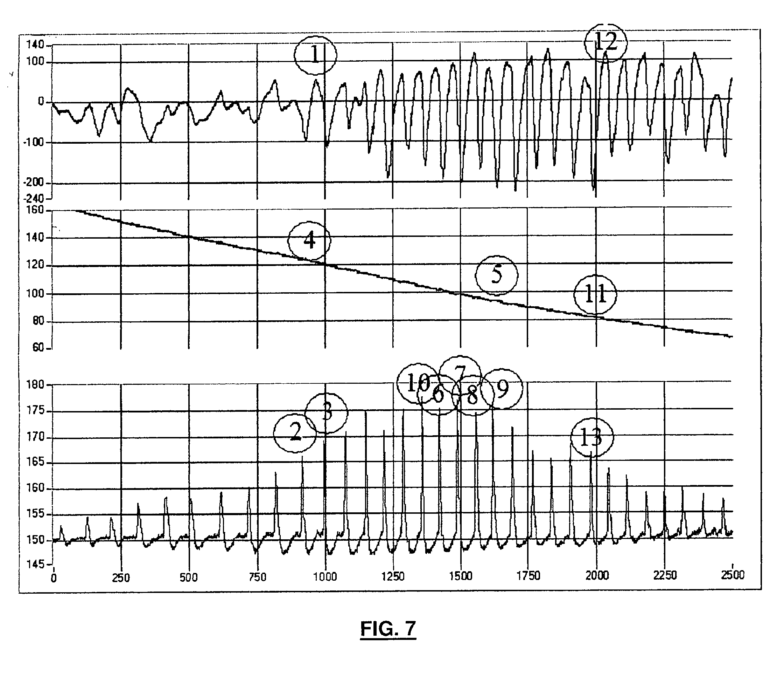

Figure 7 shows three graphs obtained by recording the signals from a cuff and a sensor, both located on a finger.
- At the top, there is the plethysmographic palpatory signal obtained with the sensor 24, of the type with a photo-plethysmographic probe;
- in the middle, there is the signal relating to the pressure on deflation of the cuff 21, obtained by the pressure transducer 22;
- at the bottom, there is the classic oscillometric signal, obtained by the oscillometric sensor 28.

In the following there are described the customised process for the self-tuning of the oscillometric method by means of a direct comparison with the auscultatory method, the particular oscillometric measurement process, and the use of the sensor fusion technique, all of which form the object of the invention and are implemented using the above-described device.

The self-tuning method is based on the simultaneous or deferred performance, preferably by a qualified operator, of the pressure measurement using the two methods, i.e. the auscultatory method, which is assumed as the "sample", and the oscillometric method. The auscultatory (or "indirect") measurement can be replaced by a "direct" measurement obtained by means of an endovascular transducer of known type.

For the self-tuning of the system for arm measurements, the operator uses a phono-endoscope to listen to the sounds produced during the process, i.e. the sounds coming from the humeral artery, and communicates the moment when the systolic pressure (Korotkoff's first sound) and diastolic pressure (Korotkoff's 4th or 5th sounds) are reached in the cuff to the system, in real time, by means of the interface keys 29. The pressure values are then stored.

The programme identifies the oscillometric peaks recorded by the device, which correspond to the previously-saved systolic and diastolic pressures, it evaluates their entity and calculates their relationship to the amplitude of the maximum oscillometric peak. The two ratios constitute the individual parameters for a given patient, which can be recalled and used by the system in subsequent oscillometric measurements.

The parameters are saved along with a previously-memorised code that is selected either automatically by the device or manually by the operator. Patients simply have to remember their own personal codes and input them before starting an automatic oscillometric measurement process.

In conventional devices, the ratios used serve as constants of the appliance common to all individuals, whereas in the device forming the object of the present invention, these ratios are strictly individual and characterise the correlation between the auscultatory and oscillometric measurements relating to the physiology of the system being measured, i.e. of a given patient.

The self-tuning of finger recordings follows the same procedure, except that the process takes place in two separate stages. First a measurement is obtained using the auscultatory method, and the patient's systolic and diastolic pressure values are saved. Then an oscillometric measurement is obtained, after which the amplitudes of the systolic and diastolic peaks are determined, based on the previously-memorised pressure values, by comparison with the amplitude of the maximum peak.

These ratios constitute two new individual parameters that are saved in the device's memory, together with the code that identifies the patient, and then reused during subsequent measurements.

The two steps described above can naturally be reversed in time. During the measurement, moreover, the recording obtained from the arm, or from the finger, may be considered alone, or in combination.

The particular features of the self-tuning method is unaffected by any pressure differences recordable between the arm and the finger, since it refers instead to values that will always be measured in the humeral artery.

In one type of standard oscillometric measurement, i.e. obtained using the arm cuff alone, it becomes necessary to preselect the individual code that identifies the specific parameters obtained during the validation process and assumed to characterise the correlation existing between the two (auscultatory and oscillometric) methods for a given individual.

The algorithm identifies the maximum oscillometric peak corresponding to the mean pressure, and then, based on the previously-saved ratios, it identifies the peaks of the oscillometric diagram corresponding to said ratios, which enable the systolic and diastolic pressures to be determined.

In alternative types of measurement, with a view to improving the robustness of the measurement and in the case of a weak pulse, as recorded in small animals (from 5 to 1.5 kg) and in paediatrics, a further oscillometric signal can be obtained, either from the arm or from a finger and/or by digital palpation, using an algorithm to solve and interpret the data according to the sensor fusion approach.

With reference to figures 5, 6 and 7, the systolic pressure (item 4) is derived in such cases by interpolating the peaks 2 and 3 of the oscillometric signal, which contain the first plethysmographic palpatory peak 1.

The mean pressure (item 5) is derived, using the classic method, in line with the maximum peak.

The minimum pressure (item 11) is sought between the first peak 12 - among the highest peaks of the palpatory signal - and the peak 13 preceding it in the oscillometric signal.

Alternatively, algorithms can be used that tend to "weigh" the different information with a view to optimising the sensor fusion method. In a more advanced system, a keypad can be included to enable the personal identification codes to be customised, or for inputting a patient's name. Using a two-key system, the procedure for inputting a name can be done in advance by specialist personnel.

Variants of the above-described non-limiting embodiments are feasible without departing from the scope of the present invention, as protected by this patent, including all equivalent embodiments for a person skilled in the art.

In particular, the customised self-validation method may be used to guarantee the "referability" to a reference method, such as the auscultator method, in all continuous arterial pressure monitoring systems that include the viewing, recording or saving of the wave representing the sphygmic signal.

During the step for the customised self-validation by comparison with the sample method, be it direct or indirect, the operator simply has to set the systolic pressure values to the maximum, and the diastolic pressure values to the minimum plethysmographic signal generated by a peripheral distal probe, such as on a finger.

This gives rise to a wave similar to the pressure wave, coinciding in line with the maximum and minimum values, with the same frequency and form defined by means of the physiological link existing, in every individual, between the pressure quantity and the plethysmographically-identified quantity of the local variation in volume.

Among the advantages of the present invention, there are various other options to choose from when conducting the measurement process:
a) the use of the auscultatory and oscillometric methods during the customised self-tuning of the oscillometric method with an arm cuff, in order to estimate the correlation between the two methods;
b) the use of the auscultatory and oscillometric methods during the customised self-tuning of the oscillometric method with a finger cuff, in order to estimate the correlation between the two methods;
c) the use of the auscultatory measurement method alone, saving the pressure values;
d) the use of the customised oscillometric measurement method alone, with an arm cuff, which is the standard option;
e) the use of the customised oscillometric measurement method alone, with a finger cuff;
f) the use of the sensor fusion method to obtain the measurement;
g) the use of the direct and oscillometric methods during the validation of the oscillometric method with the cuff applied to an animal, in order to estimate the correlation between the two methods.

From the description given above, a person skilled in the art is capable of realising the object of the invention without the need to provide further structural details.

## Claims

1. A device for measuring arterial pressure involving a customised self-validation of the measurement system, comprising:
- at least one cuff (21), applicable to the arm and/or finger, or to an animal's limb or tail;
- means for directly or indirectly recording the systolic and diastolic arterial pressure and the oscillometric signal coming from said at least one arm cuff (21) or finger cuff,
- means for saving the minimum, mean and maximum arterial pressure values for a given individual in said at least one cuff (21) during the deflation or inflation phases;
- means for determining the peak oscillometric values coinciding with said minimum and maximum arterial pressure values, and the maximum oscillometric peak value, and for determining and saving individual parameters given by the ratios between said minimum and maximum arterial pressure values and said maximum oscillometric peak value.

2. A device for measuring arterial pressure according to claim 1, comprising:
- means for identifying the maximum oscillometric peak value, which correspond to the mean pressure value;
- means for determining and calculating the systolic and diastolic pressure values based on the identified peaks of the oscillometric values, which correspond to said individual parameters.

3. A device for measuring arterial pressure according to claim 1, also comprising:
- at least one plethysmographic palpatory signal sensor (24) applicable to a finger;
- means for recording the palpatory signal coming from said plethysmographic palpatory signal (sensor 24).

4. A device for measuring arterial pressure according to claim 3, comprising means for calculating the minimum, mean and maximum arterial pressure values in order to determine:
- the maximum pressure (4) by means of an interpolation of the peaks (2, 3) of the oscillometric signal, which contain the first peak (1) of the plethysmographic palpatory signal;
- the mean pressure (5) coinciding with the peak of maximum amplitude (10) of the oscillometric signal;
- the minimum pressure (11), to be found between the first (12) of the highest peaks of the plethysmographic palpatory signal and the peak (13) that precedes it in the oscillometric signal.

5. A device for measuring arterial pressure according to claim 3 or 4, wherein said plethysmographic palpatory signal sensor (24) is of the type with an electret membrane, or with an electromagnetic film, or a resistive probe, or a piezoelectric probe, or a photo-plethysmographic probe, or a probe connected to a common pressure transducer by means of a transmission fluid.

6. A device for measuring arterial pressure according to claim 1 or 2, wherein a further elastic probe is inserted inside or downstream from said at least one cuff (21), and always in contact therewith, that contains a fluid serving the purpose of facilitating the transmission of the oscillometric signal, and that is connected to a pressure transducer for measuring said signal.

7. A device for measuring arterial pressure according to claim 1, wherein said means for recording the signal comprise:
- a micro-compressor (23) for said cuff (21);
- a manually-adjustable or fixed deflation valve (25) for said cuff (21);
- a deflation solenoid valve (6) for rapidly releasing the pressure inside said cuff (21);
- an arterial pressure sensor (22) based on the recording of the inflation-deflation pressure in said cuff (21);
- a further pressure transducer, or oscillometric sensor (28), for recording the oscillometric signal from said cuff (21);
- a microcontroller (27) for controlling the operation of the device.

8. A device for measuring arterial pressure according to claim 7, wherein said means for recording the signal further comprise:
- an amplifier-filter system for determining oscillograms;
- a display for viewing the processed data.

9. A method for the customised self-validation of the measurement system and for measuring arterial pressure using a device according to any of the previous claims, comprising the following steps:
- providing at least one cuff (21) on an arm or finger, or on the paw or tail of an animal;
- recording the arterial pressure using a direct or indirect method and simultaneously or subsequently recording the oscillometric signal coming from said at least one cuff (21);
- storing the minimum, mean and maximum arterial pressure values for a given subject during the of deflation or inflation phases in said at least one cuff (21);
- determining the peak oscillometric values coinciding with said minimum and maximum arterial pressure values, and the maximum oscillometric peak value;
- determining and storing individual parameters given by the ratios between the peaks that correspond to said minimum and maximum arterial pressure values and said maximum oscillometric peak value.

10. A method for measuring arterial pressure according to claim 9, further comprising the following steps:
- identifying the maximum oscillometric peak corresponding to the mean pressure value;
- determining the systolic and diastolic pressure values based on the identification of the peak oscillometric values corresponding to said previously-saved individual parameters.

11. A method for measuring arterial pressure according to claim 10, further comprising the following steps:
- providing at least one plethysmographic palpatory signal sensor (24) on a finger;
- recording the palpatory signal coming from said plethysmographic palpatory signal sensor (24).

12. A method for measuring arterial pressure according to claim 11, comprising further steps for:
- determining the maximum pressure (4) by means of an interpolation of the peaks (2, 3) of the oscillometric signal, which contain the first peak (1) in the plethysmographic palpatory signal;
- determining the mean pressure (5), which corresponds to the peak of maximum amplitude (10) of the oscillometric signal;
- determining the minimum pressure (11), which can be found between the first (12) among the highest peaks of the plethysmographic palpatory signal, and the peak (13) that precedes it in the oscillometric signal.

13. A method for measuring arterial pressure according to any of the claims from 9 to 13, comprising a further step for the automatic continuous measurement of the sphygmic wave by means of a continuous monitoring of the plethysmographic palpatory signal.
